# EUROPÄISCHE PATENTSCHRIFT

(11) **EP 0 842 637 B1**
(45) Veröffentlichungstag und Bekanntmachung des Hinweises auf die Patenterteilung: **25.06.2003**
(21) Anmeldenummer: 97203431.8
(22) Anmeldetag: 05.11.1997
(51) Int. Cl.: A61B 6/08

(54) **Verfahren zur Steuerung eines koppelstangenlosen Tomographiegerätes**
Control method for tomography apparatus without a coupling rod
Méthode de commande pour un appareil de tomographie sans tige de couplage

(30) Priorität: 15.11.1996 DE 19647243
(43) Veröffentlichungstag der Anmeldung: 20.05.1998
(73) Patentinhaber: Philips Intellectual Property & Standards GmbH, 20099 Hamburg (DE); Koninklijke Philips Electronics N.V., 5621 BA Eindhoven (NL)
(72) Erfinder: Biermann, Peter, Röntgenstrasse 24, 22335 Hamburg (DE); Pfeiffer, Wilfried, Röntgenstrasse 24, 22335 Hamburg (DE)
(74) Vertreter: Volmer, Georg, Dipl.-Ing.

(56) Entgegenhaltungen:
- DE-A- 4 008 778
- FR-A- 2 281 741
- US-A- 4 442 533

## Beschreibung

Die Erfindung bezieht sich auf ein Verfahren zur Steuerung der Bewegungen eines Strahlers und eines Stahlendetektors, z.B. einer Filmkassette, in bezug auf eine eingestellte Schichtlage, z.B. Schichthöhe, bei einem koppelstangenlosen Tomographiegerät mit einem Tisch zur Aufnahme eines zu tomographierenden Organs. Die Erfindung bezieht sich ferner auf eine Anordnung zur Durchführung des Verfahrens.

In der Röntgendiagnostik wird seit Jahrzehnten Tomographie mittels mechanischer Kopplung von Strahler und Film realisiert. Mit Einführung koppelstangenloser (elektronischer) Tomographie entfällt der mechanische Drehpunkt, der bislang für die Anbringung eines Lichtzeigers zur Markierung der eingestellten Schichtlage auf dem Patienten genutzt wurde. Da dieser Punkt für die Anbringung eines Lichtzeigers bei der elektronischen Tomographie nicht mehr existiert, gibt es Schwierigkeiten für die Röntgenassistentin, die genaue Schicht am Patienten einzustellen.

Durch die EP 0 054 798 B1 ist eine Justiereinheit für ein Strahlungs-Tomographiegerät bekannt geworden. Diese Bauart enthält eine auf einer Linie liegende Lichtstrahl-Detektoreinrichtung und einen Lichtstrahl-Erzeuger, wobei die horizontale Linie durch die Mitte eines Bestrahlungsbereiches verläuft. Das zu bestrahlende Objekt liegt auf einem höhenverstellbar angeordneten Tisch, der mit einer Antriebseinrichtung zum Anheben und Absenken versehen ist. Die bei der Höhenverstellung des Tisches anfallenden Daten werden elektronisch erfaßt, wobei eine Regeleinrichtung aus den erfaßten Daten ein Vergleichssignal berechnet und speichert, das zum Ausrichten der horizontalen Linie des Fotografierbereiches mit der Mitte des zu fotografierenden Teiles des Objektes notwendig ist.

Der Erfindung liegt die Aufgabe zugrunde, ein einfaches und zuverlässiges Verfahren der eingangs genannten Art zu schaffen.

Diese Aufgabe wird gemäß der Erfindung dadurch gelöst,
- daß ein am Tisch verstellbar angeordneter Lichtzeiger auf die zu tomographierende Schicht des Organgs eingestellt wird,
- daß die eingestellte Schichtlage gemessen wird,
- daß die gemessenen Daten über die Schichtlage in eine Systemsteuereinheit eingegeben werden und
- daß der Strahler und der Strahlendetektor in Abhängigkeit von den eingegebenen Daten auf die gemessene Schichtlage eingestellt werden.

Ein derartiges Verfahren ist einfach durchzuführen. Der Lichtzeiger kann problemlos von Hand eingestellt werden. Ferner kann die auf diese Weise eingestellte Lage der Schicht problemlos und schnell auf einer Skala abgelesen und z.B. von Hand in die Systemsteuereinheit eingegeben werden. Diese Systemsteuereinheit bewirkt dann die für diese Schichtlage erforderlichen, aufeinander abgestimmten Strahler- und Strahlendetektorbewegungen. Vorteilhaft bei diesem Verfahren ist, daß dabei keine Verstellung des Tisches nötig ist. Die gemessenen Daten über die eingestellte Schichtlage werden vielmehr zu der Systemsteuereinheit geleitet, die die erforderlichen Bewegungen des Strahlers und des Strahlendetektors steuert.

Die Einstellung der Schichtlage kann auf einfache Weise entweder von Hand oder motorisch erfolgen. Als Strahlendetektor kann z.B. auch ein flacher Digitaldetektor vorgesehen sein.

Ein Verfahren gemäß Anspruch 2 kann schnell, problemlos und einfach durchgeführt werden.

Eine komfortable Ausgestaltung der Erfindung ist in Anspruch 3 beschrieben. Damit kann der Arbeitsaufwand auf ein Minimum reduziert werden.

Eine erfindungsgemäße Anordnung zur Durchführung des oben genannten Verfahrens ist gekennzeichnet
- durch einen in der Nähe des zu tomographierenden Organs an dem Tisch angebrachten Halter mit einem eingebauten, verstellbar angeordneten Schieber,
- durch einen am Schieber angebrachten Lichtzeiger,
- durch Mittel zur Erfassung der Daten über die Schichtlage und
- durch eine Systemsteuereinheit zur Verarbeitung der Daten über die Schichtlage und zur Weiterleitung der Daten an ein Gerät zur Steuerung der Bewegung des Strahlers und des Strahlendetektors.

Eine derartige Anordnung ermöglicht mit wenigen Bauteilen eine zuverlässige, schnell durchführbare Markierung der eingestellten Schichtlage auf dem Patienten und eine entsprechend ausgerichtete Lage des Strahlers und des Strahlendetektors.

Eine gute Markiermöglichkeit ergibt sich dadurch, daß als Lichtzeiger ein Laserlichtzeiger vorgesehen ist.

Die Merkmale des Anspruches 6 ermöglichen eine schnelle Eingabe der Daten, wenn die Daten von der betreuenden Person selbst von der Skala abgelesen werden.

Eine Vereinfachung ist gekennzeichnet durch eine Einrichtung zur automatischen Erfassung und Weiterleitung der elektronisch gemessenen Daten über die Schichtlage.

Der Halter selbst kann in weiterer Ausgestaltung der Erfindung abnehmbar oder wegklappbar angebracht sein. Dadurch wird die Arbeitsfläche des Tisches nicht behindert, wenn keine Einstellung der Schichtlage erforderlich ist.

In Ausgestaltung der Erfindung ist vorgesehen, daß bei elektronischer Messung, Erfassung und Weiterleitung der Daten über die Schichtlage die Signalübermittlung entweder über Kabel oder drahtlos, z.B. per Funk oder Ultraschall, erfolgt.

Eine einfache Ausführung der Erfindung ist gekennzeichnet durch einen im Halter angebrachten Akku für die Lichtzeiger-Spannungsversorgung.

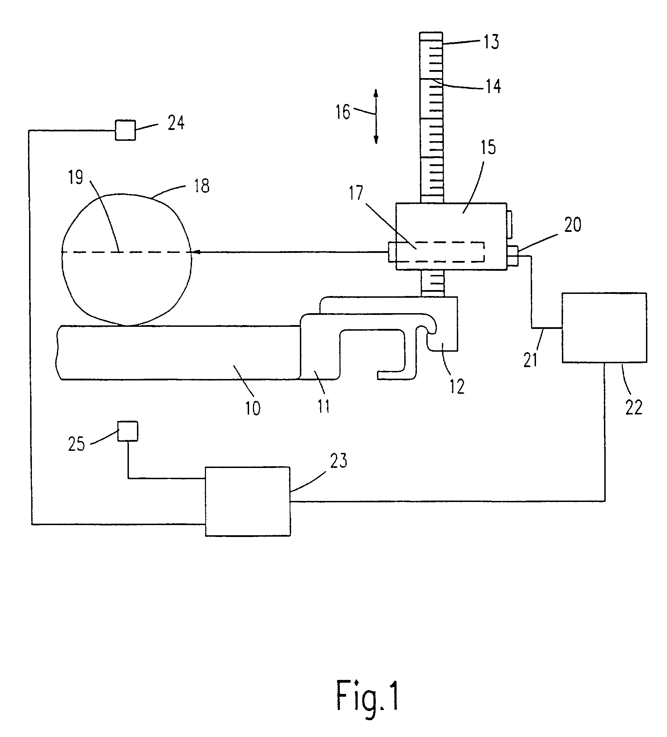

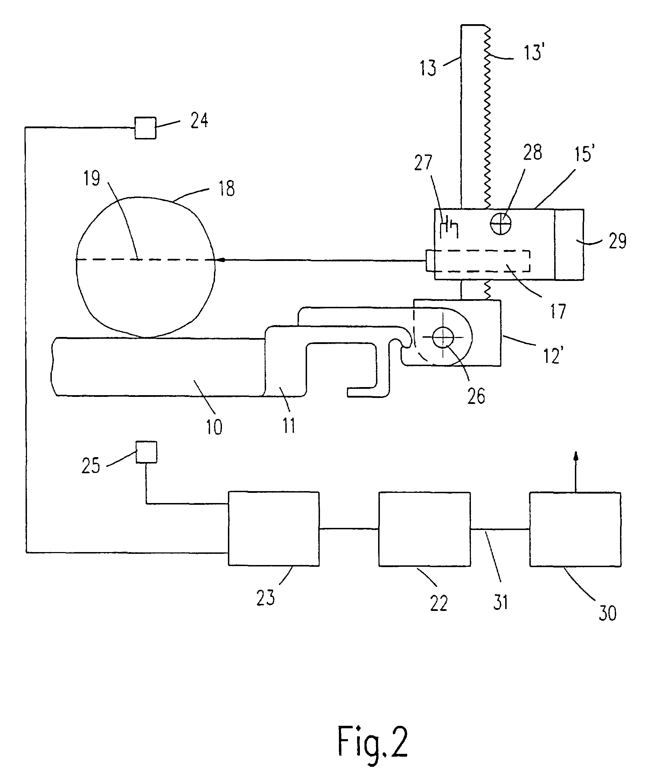

In der Zeichnung ist in Fig. 1 und 2 ein Ausführungsbeispiel des Gegenstandes gemäß der Erfindung schematisch dargestellt.
Fig. 1 zeigt einen Teil eines Tisches mit einem einen Laserzeiger aufweisenden Halter, und
Fig. 2 zeigt eine spezielle Gestaltung des Laserzeigers bei einem etwas abgeänderten Halter.

Fig. 1 zeigt einen teilweise dargestellten Tisch 10 eines Tomographiegerätes mit einem im Tisch-Randprofil 11 abnehmbar angebrachten Halter 12. Der Halter 12 besitzt eine Leiste 13 mit einer darauf angebrachten Skala 14. Auf der Leiste 13 ist ein Schieber 15 in Richtung 16 vertikal beweglich angeordnet. Der Schieber 15 trägt einen Laserlichtzeiger 17, mit dessen Hilfe bei einem auf dem Tisch 10 liegenden Organ 18 eine bestimmte Schichthöhe 19 eingestellt werden kann. Der Schieber 15 kann mit Hand oder motorisch auf der Leiste 13 verstellt werden. Die mit Hilfe des Schiebers 15 eingestellte Schichthöhe 19 kann gemäß Fig. 1 auf der Skala 14 abgelesen werden. Die abgelesenen Daten können sodann bei der Bauart gemäß Fig. 1 von Hand in einen Steuergriff 20 eingegeben werden und werden von dort über ein Kabel 21 in eine Systemsteuereinheit 22 weitergeleitet. Die Systemsteuereinheit 22 steht mit einem Steuergerät 23 in Verbindung, welches aufgrund der empfangenen Daten einen Strahler 24 und als Strahlendetektor eine Filmkassette 25 auf die eingestellte Schichthöhe 19 ausrichtet.

Gemäß Fig. 2 ist ein klappbarer Halter 12' vorgesehen, der um den Drehpunkt 26 seitlich weggeschwenkt werden kann. Diese Bauart zeigt ferner einen Schieber 15', der einen Akku 27 für die Lichtzeiger-Spannungsversorgung enthält. Mit 28 ist ein elektronisches Meßgerät zur elektronischen Messung der eingestellten Höhenlage bezeichnet. Diese elektronisch erfaßten Meßdaten werden sodann über einen im Schieber 15' enthaltenen Sender 29 drahtlos zu einem Empfägner 30 geleitet, der die entsprechenden Daten über die Höhenlage über Kabel 31 zu der Systemsteuereinheit 22 führt. Von hier aus gelangen die Daten sodann wieder zu dem Steuergerät 23 zur Steuerung des Strahlers 24 und der Filmkassette 25. Mit 13' ist eine Zahnstange angedeutet.

## Patentansprüche

1. Verfahren zur Steuerung der Bewegungen eines Strahlers (24) und eines Strahlendetektors, z.B. einer Filmkassette (25), in bezug auf eine eingestellte Schichtlage, z.B. Schichthöhe (19), bei einem koppelstangenlosen Tomographiegerät mit einem Tisch (10) zur Aufnahme eines zu tomographierenden Organs (18),
**dadurch gekennzeichnet,**
- **daß** ein am Tisch (10) verstellbar angeordneter Lichtzeiger (17) auf die zu tomographierende Schicht des Organs (18) eingestellt wird,
- **daß** die eingestellte Schichtlage (19) gemessen wird,
- **daß** die gemessenen Daten über die Schichtlage in eine Systemsteuereinrichtung (22) eingegeben werden und
- **daß** der Strahler (24) und der Strahlendetektor (25) in Abhängigkeit von den eingegebenen Daten auf die gemessene Schichtlage (19) eingestellt werden.

2. Verfahren nach Anspruch 1, **dadurch gekennzeichnet, daß** die eingestellte Schichtlage an einer Skala (14) abgelesen wird und daß die an der Skala (14) abgelesenen Daten über die Schichtlage von Hand in die Systemsteuereinheit (22) eingegeben werden.

3. Verfahren nach Anspruch 1, **dadurch gekennzeichnet, daß** die eingestellte Schichtlage elektronisch gemessen wird und daß die Meßdaten automatisch erfaßt und an die Systemsteuereinheit (22) weitergeleitet werden.

4. Anordnung zur Durchführung des Verfahrens nach einem der Ansprüche 1 bis 3, **gekennzeichnet**
- **durch** einen in der Nähe des zu tomographierenden Organs (18) an dem Tisch (10) angebrachten Halter (12, 12') mit eingebautem, verstellbar angeordnetem Schieber (15, 15'),
- **durch** einen am Schieber (15, 15') angebrachten Lichtzeiger (17),
- **durch** Mittel (14, 28) zur Erfassung der Daten über die Schichtlage und
- **durch** eine Systemsteuereinheit (22) zur Verarbeitung der Daten über die Schichtlage und zur Weiterleitung der Daten an ein Gerät (23) zur Steuerung der Bewegungen des Strahlers (24) und des Strahlendetektors (25).

5. Anordnung nach Anspruch 4, **dadurch gekennzeichnet, daß** als Lichtzeiger ein Laserlichtzeiger (17) vorgesehen ist.

6. Anordnung nach Anspruch 4, **gekennzeichnet durch** eine elektronische Vorrichtung (28) zur Messung der jeweils eingestellten Schichtlage.

7. Anordnung nach Anspuch 4, **gekennzeichnet durch** einen Steuergriff (20) zur Handeingabe der von einer Skala (14) abgelesenen Daten über die Schichtlage in die Systemsteuereinheit (22).

8. Anordnung nach Anspruch 4, **gekennzeichnet durch** eine Einrichtung (28) zur automatischen Erfassung und Weiterleitung der elektronisch gemessenen Daten über die Schichtlage.

9. Anordnung nach Anspruch 4, **dadurch gekennzeichnet, daß** der Halter (12, 12') abnehmbar oder wegklappbar ausgebildet ist.

10. Anordnung nach Anspruch 6, **dadurch gekennzeichnet, daß** bei einer elektronischen Messung, Erfassung und Weiterleitung der Daten über die Schichtlage die Systemsteuereinrichtung (22) entweder über Kabel oder drahtlos, z.B. per Funk oder Ultraschall, erfolgt.

11. Anordnung nach Anspruch 5, **gekennzeichnet durch** einen im Schieber (15, 15') angebrachten Akku (27) für die Lichtzeiger-Spannungsversorgung.

## Claims

1. A method of controlling the motions of a radiation source (24) and a radiation detector, for example, a film cassette (25), in relation to an adjusted slice position, for example, a slice level (19), in a tomography apparatus without a coupling rod, which tomography apparatus includes a table (10) for accommodating an organ (18) to be imaged by tomography, **characterized in that**:
- a light indicator (17) which is adjustably mounted on the table (10) is adjusted to the slice of the organ (18) to be imaged by tomography,
- the adjusted slice position (19) is measured,
- the measured slice position data is applied to a system control unit (22), and
- the source (24) and the radiation detector (25) are adjusted in relation to the measured slice position (19) in conformity with the data applied.

2. A method as claimed in claim 1, **characterized in that** the adjusted slice position is read from a scale (14) and that the slice position data read from the scale (14) is applied to the system control unit (22) by hand.

3. A method as claimed in claim 1, **characterized in that** the adjusted slice position is electronically measured and that the measurement data is automatically acquired and applied to the system control unit (22).

4. A device for carrying out the method claimed in one of the claims 1 to 3, **characterized in that** it includes:
- a holder (12, 12') which accommodates a built-in adjustable slider (15, 15') and is mounted on the table (10) in the vicinity of the organ (18) to be imaged,
- a light indicator (17) which is mounted on the slider (15, 15'),
- means (14, 28) for the acquisition of the slice position data, and
- a system control unit (22) for processing the slice position data and for applying the data to an apparatus (23) for controlling the motions of the source (24) and the radiation detector (25).

5. A device as claimed in claim 4, **characterized in that** the light indicator is a laser light indicator (17).

6. A device as claimed in claim 4, **characterized in that** it includes an electronic device (28) for measuring the instantaneously adjusted slice position.

7. A device as claimed in claim 4, **characterized in that** it includes a control grip (20) for applying the slice position data read from a scale (14) to the system control unit (22) by hand.

8. A device as claimed in claim 4, **characterized in that** it includes a device (28) for the automatic acquisition and input of the electronically measured slice position data.

9. A device as claimed in claim 4, **characterized in that** the holder (12, 12') is constructed in such a manner that it can be detached or swung aside.

10. A device as claimed in claim 6, **characterized in that** the signal transmission for the electronic measurement, acquisition and application of the slice position data to the system control device (22) takes place either via a cable or in a wireless manner, for example by radio or ultrasound.

11. A device as claimed in claim 5, **characterized in that** the slider (15, 15') accommodates a battery (27) for power supply of the light indicator.

## Revendications

1. Procédé de commande des mouvements d'un générateur de rayons X (24) et d'un détecteur de rayons X, par exemple d'une cassette de film (25) par rapport à une position de couche réglée, p. ex. une hauteur de couche (19) en cas d'appareil de tomographie sans tige de couplage avec une table (10) pour loger un organe (18) à tomographier,
**caractérisé en ce**
- **qu'**un pointeur lumineux (17) disposé à déplacement sur la table (10) est réglé sur la couche à tomographier de l'organe (18),
- **que** la position de couche (19) réglée est mesurée,
- **que** les données mesurées sur la position de couche sont introduites dans un dispositif de commande du système (22) et que
- le générateur de rayons X (24) et le détecteur de rayons X (25) sont réglés en fonction des données introduites sur la position de couche (19) mesurée.

2. Procédé selon la revendication 1, **caractérisé en ce que** la position de couche réglée est lue sur une échelle (14) et que les données lues sur l'échelle (14) à propos de la position de couche sont introduites à la main dans l'unité de commande du système (22).

3. Procédé selon la revendication 1, **caractérisé en ce que** la position de couche réglée est mesurée électroniquement et que les données de mesure sont saisies automatiquement et transmises à l'unité de commande du système (22).

4. Dispositif de mise en oeuvre du procédé selon l'une des revendications 1 à 3, **caractérisé**
- **par** un support (12, 12') disposé sur la table (10) à proximité de l'organe à tomographier (18) avec un tiroir intégré, disposé de manière réglable (15, 15') ;
- par un pointeur lumineux (17) placé sur le tiroir (15, 15');
- par des moyens (14, 28) pour la saisie des données à propos de la position de couche et
- par une unité de commande du système (22) pour le traitement des données sur un appareil (23) pour la commande des mouvements du générateur de rayons X (24) et du détecteur de rayons X (25).

5. Dispositif selon la revendication 4, **caractérisé en ce qu'**un pointeur lumineux laser (17) est prévu comme pointeur lumineux.

6. Dispositif selon la revendication 4, **caractérisé par** un dispositif électronique (28) de mesure de la position de couche respectivement réglée.

7. Dispositif selon la revendication 4, **caractérisé par** une poignée de commande (20) pour l'introduction manuelle des données lues par une échelle (14) à propos de la position de couche dans l'unité de commande du système (22).

8. Dispositif selon la revendication 4, **caractérisé par** un dispositif (28) pour la saisie automatique et le transfert de données mesurées électroniques à propos de la position de couche.

9. Dispositif selon la revendication 4, **caractérisé en ce que** le support (12, 12') est de conception amovible ou repliable.

10. Dispositif selon la revendication 6, **caractérisé en ce qu'**en cas de mesure, saisie et transfert électroniques des données à propos de la position de couche, le dispositif de commande du système (22) fonctionne soit par câble ou sans fil, p. ex. par radio ou ultrasons.

11. Dispositif selon la revendication 5, **caractérisé par** un accumulateur (27) placé dans le tiroir (15, 15') pour l'alimentation en tension du pointeur lumineux.
